# EUROPEAN PATENT SPECIFICATION

(11) **EP 0 648 783 B1**
(45) Date of publication and mention of the grant of the patent: **29.03.2000**
(21) Application number: 94116015.2
(22) Date of filing: 11.10.1994
(51) Int. Cl.: C07K 16/28, C12N 15/13, C12N 15/62, C12P 21/08, A61K 39/395

(54) **TNF inhibitors**
TNF-Inhibitoren
Inhibiteurs de TNF

(30) Priority: 12.10.1993 IL 10726793
(43) Date of publication of application: 19.04.1995
(73) Proprietor: YEDA RESEARCH AND DEVELOPMENT CO. LTD., Rehovot 76100 (IL)
(72) Inventor: Wallach , David, Rehovot (IL); Bigda , Jacek, 80-809 Gdansk (PL); Beletsky , Igor, RAS Puschino 142292 (RU); Mett, Igor, Rehovot (IS)
(74) Representative: VOSSIUS & PARTNER

(56) References cited:
- EP-A- 0 398 327
- EP-A- 0 412 486
- EP-A- 0 585 939
- J.BIOL.CHEM., vol.267, no.9, 1992, USA pages 5747 - 5750 S.A.MARSTERS ET AL. 'Identification of Cysteine-rich Domains of the Type 1 Tumor Necrosis Factor Receptor Involved in Ligand Binding'

## Description

The present invention relates to ligands to Tumor Necrosis Factor receptors (TNF-Rs) which inhibit the effect of TNF but not its binding to the TNF-Rs, as well as to ligands interacting with other receptors of the TNF/NGF family.

Tumor necrosis factor (TNF) is a pleiotropic cytokine, produced by a number of cell types, mainly by activated macrophages. It is one of the principal mediators of the immune and inflammatory response. Interest in its function has greatly increased, recently, in view of evidence of the involvement of TNF in the pathogenesis of a wide range of disease states, including endotoxin shock, cerebral malaria and graft-versus-host reaction. Since many of the effects of TNF are deleterious to the organism, it is of great interest to find ways of blocking its action on host cells. An evident target for such intervention are the molecules to which TNF has to bind in order to exert its effects, namely the TNF-Rs. These molecules exist not only in cell-bound, but also in soluble forms, consisting of the cleaved extra-cellular domains of the intact receptors (see Nophar et al., EMBO Journal, 9(10):3269-78, 1990). The soluble receptors maintain the ability to bind TNF, and thus have the ability to block its function by competition with surface receptors.

Another method of TNF inhibition based on the principle of competing with cell-bound molecules, is the use of antibodies recognizing TNF receptors and blocking the ligand binding.

The cell surface TNF-Rs are expressed in almost all cells of the body. The various effects of TNF, the cytotoxic, growth-promoting and others, are all signalled by the TNF receptors upon the binding of TNF to them. Two forms of these receptors, which differ in molecular size: 55 and 75 kilodaltons, have been described, and will be called herein p55 and p75 TNF-R, respectively. It should be noted, however, that there exist publications which refer to these receptors also as p60 and p80.

The TNF-Rs belong to a family of receptors which are involved in other critical biological processes. Examples of these receptors are the low affinity NGF receptor, which plays an important role in the regulation of growth and differentiation of nerve cells. Several other receptors are involved in the regulation of lymphocyte growth, such as CDw40 and some others. Another member of the family is the FAS receptor also called APO, a receptor which is involved in signalling for apoptosis and which, based on a study with mice deficient in its function, seems to play an important role in the etiology of a lupus-like disease. Herein, this family of receptors is called "TNF/NGF receptor family".

One of the most striking features of TNF compared to other cytokines, thought to contribute to the pathogenesis of several diseases, is its ability to elicit cell death. The cell-killing activity of TNF is thought to be induced by the p55 receptor. However, this p55 receptor activity can be assisted by the p75 receptor, through a yet unknown mechanism.

European patent application No. EP-A1-0 412 486 discloses antibodies to the soluble TNF-Rs. These antibodies were found to recognize the soluble TNF-Rs and to inhibit the binding of TNF to the TNF-Rs on the cell surface. Monovalent F(ab) fragments blocked the effect of TNF, while intact antibodies were observed to mimic the cytotoxic effect of TNF. European patent application No. EP-A2-0 585 939 describes ligands interacting with a certain region in TNF-Rs. Masters, J. Biol. Chem. 467 (1992), 5747-5750 describes that p55 and p75 TNF-R similar to other cell surface proteins including nerve growth factor receptors contain a repetitive amino acid sequence pattern of four cysteine rich domains.

The present invention provides a ligand to a member of the TNF/NGF receptor family, which binds either to the region of the fourth cysteine rich domain of such a receptor, or to the region between it and the cell membrane.

The region of the fourth cysteine rich domain will be called herein, for simplicity's sake, the "67 epitope" and the antibodies recognizing it the "group 67" antibodies. This region may extend between amino acids pro-141 and thr-179 in the p75 TNF-R or a corresponding region in another member of the TNF/NGF family. More particularly, the region may extend between amino acids cys-163 and pro-141 of the p75 TNF-R or a corresponding region in another member of the TNF/NGF family. The ligand downstream of the fourth cysteine rich domain includes the amino acid sequence between thr-179 and the end of the extracellular domain of the receptor or a corresponding region in another member of the TNF/NGF family.

Preferably, the receptor is the TNF-R, in particular the p75 TNF-R.

One such ligand includes the amino acid sequence for the CDR region of the heavy chain of monoclonal antibody no. 67 and/or of the light chain thereof.

Another such ligand includes the amino acid sequence for the CDR region of the heavy chain of monoclonal antibody no. 81, and/or the light chain thereof.

Yet another such ligand includes the amino acid sequence for antibody against the "stalk" region, i.e. from about amino acid thr-181 to about amino acid 235-asp.

The ligands may comprise e.g. proteins, peptides, immunoadhesins, antibodies or other organic compounds.

The proteins may comprise, for example, a fusion protein of the ligand with another protein, optionally linked by a peptide linker. Such a fusion protein can increase the retention time of the ligand in the body, and thus may even allow the ligand-protein complex to be employed as a latent agent or as a vaccine.

The term "proteins" includes muteins and fused proteins, their salts, functional derivatives and active fractions.

As used herein the term "muteins" refers to analogs of the proteins, peptides and the like in which one or more of the amino acid residues of the protein found to bind are replaced by different amino acid residues or are deleted, or one or more amino acid residues are added to the original sequence, without changing considerably the activity of the resulting product. These muteins are prepared by known synthesis and/or by site-directed mutagenesis techniques, or any other known technique suitable therefor.

The term "fused protein" refers to a polypeptide comprising the ligands or a mutein thereof fused with another protein which has an extended residence time in body fluids. The ligands may thus be fused to another protein, polypeptide or the like, e.g. an immunoglobulin or a fragment thereof.

The term "salts" herein refers to both salts of carboxyl groups and to acid addition salts of amino groups of the ligands, muteins and fused proteins thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid.

"Functional derivatives" as used herein cover derivatives of the ligands and their fused proteins and muteins, which may be prepared from the functional groups which occur as side chains on the residues or the N- or C- terminal groups, by means known in the art, and are included in the invention as long as they remain pharmaceutically acceptable, i.e. they do not destroy the activity of the ligand and do not confer toxic properties on compositions containing it. These derivatives may, for example, include polyethylene glycol side chains which may mask antigenic sites and extend the residence of the ligands in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

As "active fractions" of the ligands, its fused proteins and its muteins, the present invention covers any fragment or precursors of the polypeptide chain of the ligand alone or together with associated molecules or residues linked thereto, e.g. sugar or phosphate residues, or aggregates of the protein molecule or the sugar residues by themselves, provided said fraction has the same biological and/or pharmaceutical activity.

The peptides include peptide bond replacements and/or peptide mimetics, i.e. pseudopeptides, as known in the art (see e.g. proceedings of the 20th European Peptide Symposium, ed. G. Jung, E. Bayer, pp. 289-336), as well as salts and pharmaceutical preparations and/or formulations which render the bioactive peptide(s) particularly suitable for oral, topical, nasal spray, ocular, pulmonary, I.V. or subcutaneous delivery, depending on the particular treatment indicated. Such salts, formulations, amino acid replacements and pseudopeptide structures may be necessary and desirable to enhance the stability, formulation, deliverability (e.g. slow release, prodrugs), or to improve the economy of production, as long as they do not adversely affect the biological activity of the peptide.

Besides substitutions, three particular forms of peptide mimetic and/or analogue structures of particular relevance when designating bioactive peptides, which have to bind to a receptor while risking the degradation by proteinases and peptidases in the blood, tissues and elsewhere, may be mentioned specifically, illustrated by the following examples: Firstly, the inversion of backbone chiral centres leading to D-amino acid residue structures may, particularly at the N-terminus, lead to enhanced stability for proteolytical degradation without adversely affecting activity. An example is given in the paper "Tritriated D-ala¹-Peptide T Binding", Smith C.S. et al., Drug Development Res. 15, pp. 371-379 (1988). Secondly, cyclic structure for stability, such as N to C interchain imides and lactames (Ede et al. in Smith and Rivier (Eds.) "Peptides: Chemistry and Biology", Escom, Leiden (1991), pp. 268-270), and sometimes also receptor binding may be enhanced by forming cyclic analogues. An example of this is given in "Confirmationally restricted thymopentin-like compounds", US Pat. 4,457,489 (1985), Goldstein, G. et al. Thirdly, the introduction of ketomethylene, methylsuflide or retroinverse bonds to replace peptide bonds, i.e. the interchange of the CO and NH moieties are likely to enhance both stability and potency. An example of this type is given in the paper "Biologically active retroinverso analogues of thymopentin", Sisto A. et al in Rivier, J.E. and Marshall, G.R. (eds) "Peptides, Chemistry, Structure and Biology", Escom, Leiden (1990), pp. 722-773).

The peptides of the invention can be synthesized by various methods which are known in principle, namely by chemical coupling methods (cf. Wunsch, E: "Methoden der organischen Chemie", Volume 15, Band 1 + 2, Synthese von Peptiden, Thime Verlag, Stuttgart (1974), and Barrany, G.; Marrifield, R.B.: "The Peptides", eds. E. Gross, J. Meienhofer, Volume 2, Chapter 1, pp. 1-284, Academic Press (1980)), or by enzymatic coupling methods (cf. Widmer, F. Johansen, J.T., Carlsberg Res. Commun., Vol.44, pp. 37-46 (1979), and Kullmann, W.: "Enzymatic Peptide Synthesis" CRC Press Inc. Boca Raton, Fl. (1987), and Widmer, F., Johansen, J.T. in "Synthetic Peptides in Biology and Medicines:, eds. Alitalo, K., Partanen, P., Vatieri, A., pp.79-86, Elsevier, Amsterdam (1985)), or by a combination of chemical and enzymatic methods if this is advantageous for the process design and economy.

A cysteine residue may be added at both the amino and carboxy terminals of the peptide, which will allow the cyclisation of the peptide by the formation of a di-sulphide bond.

Any modifications to the peptides of the present invention which do not result in a decrease in biological activity are within the scope of the present invention.

There are numerous examples which illustrate the ability of anti-idiotypic antibodies (anti-Id Abs) to an antigen to function like that antigen in its interaction with animal cells and components of cells. Thus, anti-Id Abs to a peptide hormone antigen can have hormone-like activity and interact specifically with a mediator in the same way as the receptor does. (For a review of these properties see: Gaulton, G.N. and Greane, M.I. 1986. Idiotypic mimicry of biological receptors, Ann. Rev. Immunol. Vol. 4, pp. 253-280; Sege K. and Peterson, P.A., 1978, Use of anti-idiotypic antibodies as cell surface receptor probes, Proc. Natl. Acad. Sci. U.S.A., Vol. 75, pp. 2443-2447).

It is expected from this functional similarity of anti-Id Ab and antigen, that anti-Id Abs bearing the internal image of an antigen can induce immunity to such an antigen. (See review in Hiernaux, J.R., 1988, Idiotypic vaccines and infectious diseases, Infect. Immun., Vol. 56, pp. 1407-1413).

It is therefore possible to produce anti-idiotypic antibodies to the peptides of the present invention which will have similar biological activity.

Accordingly, the present invention also provides anti-idiotypic antibodies to the peptides of the present invention, the anti-idiotypic antibody being capable of inhibiting TNF toxicity, but not its binding to the receptor.

The individual specificity of antibodies resides in the structures of the peptide loops making up the Complementary Determining Regions (CDRs) of the variable domains of the antibodies. Since in general the amino acid sequence of the CDR peptides of an anti-Id Ab are not identical to or even similar to the amino acid sequence of the peptide antigen from which it was originally derived, it follows that peptides whose amino acid sequence in quite dissimilar, in certain contexts, can take up a very similar three-dimensional structure. The concept of this type of peptide, termed a "functionally equivalent sequence" or mimotope by Geyson is known. (Geyson, H.M. et al, 1987, Strategies for epitope analysis using peptide synthesis., J. Immun. Methods, Vol. 102, pp. 259-274).

Moreover, the three-dimensional structure and function of the biologically active peptides can be simulated by other compounds, some not even peptidic in nature, but which nevertheless mimic the activity of such peptides. This field is summarized in a review by Goodman, M. (1990), (Synthesis, Spectroscopy and computer simulations in peptide research, Proc. 11th American Peptide Symposium published in Peptides-Chemistry, Structure and Biology, pp. 3-29; Eds. Rivier, J.E. and Marshall, G.E. Publisher Escom).

It is also possible to produce peptide and non-peptide compounds having the same three-dimensional structure as the peptides of the present invention. These "functionally equivalent structures" or peptide mimics" will react with antibodies raised against the peptide of the present invention and may also be capable of inhibiting TNF toxicity.

Accordingly, a further embodiment of the present invention provides a compound the three-dimensional structure of which is similar as a pharmacophore to the three-dimensional structure of the peptides of the present invention, the compound being characterized in that it reacts with antibodies raised against the peptides of the present invention and that the compound is capable of inhibiting TNF toxicity.

More detail regarding pharmacophores can be found in Bolin et al., p. 150, Polinsky et al., p. 287, and Smith et al., p. 485, in Smith and Rivier (eds.) "Peptides: Chemistry and Biology", Escom, Leiden (1991).

All of the molecules (proteins, peptides, etc.) may be produced either by conventional chemical methods, as described herein, or by recombinant DNA methods.

The invention also provides DNA molecules encoding the ligands according to the invention, vectors containing them and host cells comprising the vectors and capable of expressing the ligands according to the invention.

The host cell may be either prokaryotic or eukaryotic.

The invention further provides DNA molecules hybridizing to the above DNA molecules and encoding ligands having the same activity.

The invention also provides pharmaceutical compositions comprising the above ligands which are useful for treating diseases induced or caused by the effects of TNF, either endogenously produced or exogenously administered.

The invention also provides for using the ligands according to the invention for increasing the inhibitory effect of a soluble receptor of the TNF/NGF receptor family. As stated above, the soluble receptors, especially those of TNF, have the ability to block the function of TNF by binding it in competition with the surface receptors. Application of a ligand according to the invention together with a soluble receptor is therefore expected to increase the inhibitory effect of the soluble receptor.

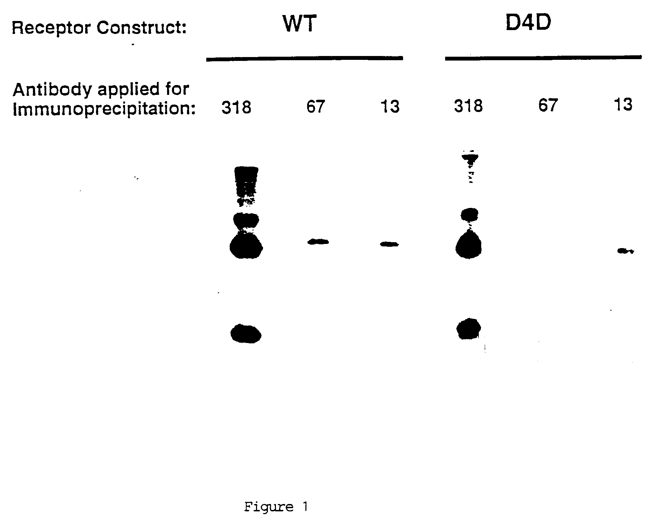

Figure 1 shows the results of the test by which epitope 67 was mapped.

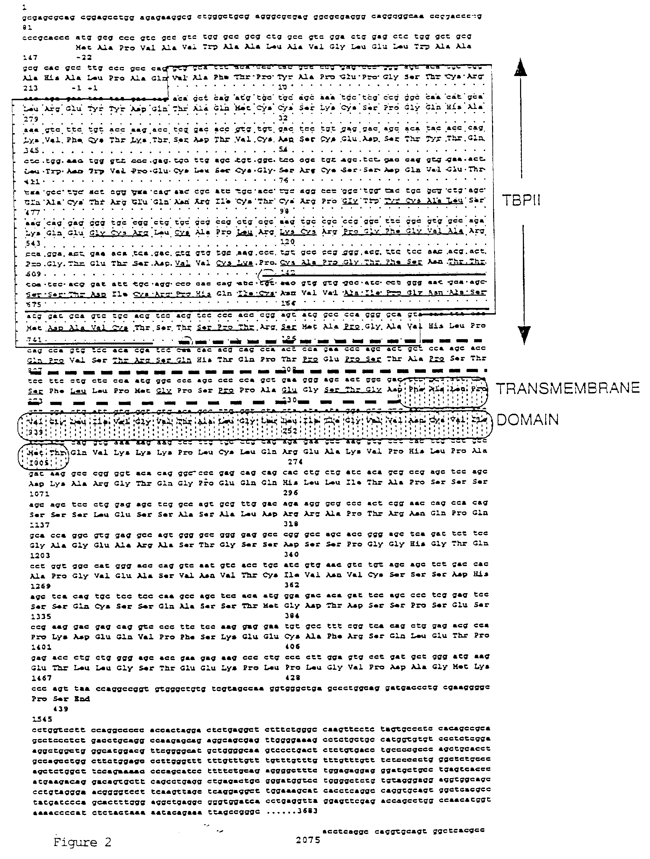

Figure 2 shows the nucleotide and deduced amino acid sequences of the p75 receptor. TBP-II and transmembranal domains are boxed and shaded. The region recognized by the group 67 antibodies is underlined, and the region recognized by the anti-stalk antibodies is underlined by a broken line.
Figure 3 shows the inhitory effect of the 67 and anti-stalk antibodies on TNF funtion in HeLa cells.
Figure 4 shows that antibodies against the upper part of extracellular domain of the p75 TNF-R are signalling in the HeLa cells.
Figure 5 shows that antibodies against the upper part of the extracellular domain of the p75 TNF-R do not signal in A9 cells which express the human p75 TNF-R. Antibodies of the 67 group do have, though, an inhibitory effect on TNF funtion in them (Fig. 6).
Figure 6 shows that antibodies against the upper part of the extracellular domain of the p75 TNF-R inhibit TNF function in A9 cells.
Figure 7 shows that antibodies against the upper part of the extracellular domain of the p75 TNF-R do not signal in A9 cells which express the cytoplasmically truncated p75 TNF-R. Antibodies of the 67 group do have, though, an inhibitory effect on TNF function in them (not shown).
Figure 8 shows that antibodies against the 67 epitope impede TNF dissociation from p75 TNF-R.
Figure 9 shows the sequence homology between several members of the TNF/NGF receptor family.

TNF, as stated above, is a cytokine which initiates its effect on cell function by binding to two specific cell surface receptors: the p55 and p75 receptors. Binding of antibodies to the extracellular domain of these receptors can interfere with its effect. However, as shown in a number of studies, antibodies binding to the extracellular domain of the receptors can also trigger the effects of TNF by inducing aggregation of the p55 receptors, as well as by inducing aggregation of the p75 receptors. (Engelmann, et al. J. Biol. Chem., Vo. 265, No. 24, pp. 14497-14504, 1990; and unpublished data).

As disclosed in European patent application No. EP-A2-0 585 939, antibodies binding to one particular region in the p75 receptor are not mimetic but rather inhibitory to the signalling for the cytocidal effect by this receptor. This, in spite of the fact that when binding to this region, these antibodies do not block TNF binding, but rather increase it to some extent. In application No. 106271 this region is more particularly identified as extending between cys-163 and thr-179, in the fourth cysteine rich domain of the receptor. The present invention reveals that the region recognized by certain other antibodies is the region extending downstream of thr-181 and upstream to cys-163 till about cys-142 in the extracellular domain of the p75 receptor.

The present invention also reveals that the so-called "stalk-antibody" recognizes a region downstream of the fourth cysteine rich domain, more particularly the region extending from about amino acid 181 to about amino acid 235.

It was also found in accordance with the present invention that, in case of the "67 epitope" antibodies, the divalent antibodies have an effect which mimics TNF action, while the monovalent fragments, such as F(ab), inhibit the cytotoxic effect of TNF.

Based on these findings, small molecular weight compounds, such as peptides or mimetic compounds, which will either inhibit the function of the p75 receptor, or enhance it, can be defined.

In view of these findings, as well as the close similarity of the receptors in this particular family, this invention relates also to ligands which bind to the same regions in the extracellular domain of the various other members of the TNF/NGF receptor family and modulate the function of the other receptors, similarly to the modulation of the function of TNF. In this receptor family, the localization of cysteines in the extra-cellular domain and the spacing is highly conserved. Certain members of this family, e.g. CDw40, exhibit particularly high similarity to the p75 receptor. Particularly in such receptors, ligands binding to these regions are expected to have effects similar to the effect of the ligands according to the present invention on the p75 receptor.

Recombinant production of the ligands is carried out by known methods commonly employed in the art.

The invention is illustrated by the following non-limiting examples:

### EXAMPLE 1: Mapping of the epitope 67 of the p75 TNF-R

a) In order to compare the function of the 67 group antibodies, not only to antibodies which bind to the receptor at the 67 epitope region, but also to antibodies that bind to the receptor downstream to that epitope region, we immunized rabbits with a chimeric construct corresponding to the region extending downstream to the 32 epitope (amino acids 181 to 235; the "stalk" region), linked to MBP. The rabbits developed antibodies which bound to the chimera with which they were immunized as well as to the intact p55 TNF receptor. These antibodies were affinity purified by binding to the chimeric protein, linked to an Affigel 10 column, and tested for effect on TNF function and binding. (The affinity purified antibody preparation was termed "318").
b) The mapping of epitope 67 was carried out by examining the ability of antibodies number 67 and 13 (an antibody that binds to the upper part of the extracellular domain of the p75 TNF-R) as well as antiserum 318, to immunoprecipitate the following methionine-labeled soluble p75 TNF-R mutants: WT- a receptor extending from amino acid 22 to amino acid 234, D4D- a receptor like WT, from which the 4th cysteine-rich domain has been deleted (amino acids 141 to 180). The receptors were produced by **in vitro** transcription of cDNAs encoding them (from the Bluescript vector, using the T7 promoter) followed by **in vitro** translation using the Promega TnT kit. The immunoprecipitated proteins were analyzed by SDS PAGE, followed by autoradiography. (A) Immunoprecipitation of WT. All antibodies were effective. (B) Immunoprecipitation of D4D. Only antibodies 13 and 318 were effective. The findings indicate that epitope 67 is located at the upper part of the 4th cysteine rich domain, within about amino acids 141 to 180.

### EXAMPLE 2: Titration of the inhibitory effect of the goup 67 antibodies and the anti-stalk antibodies on TNF function

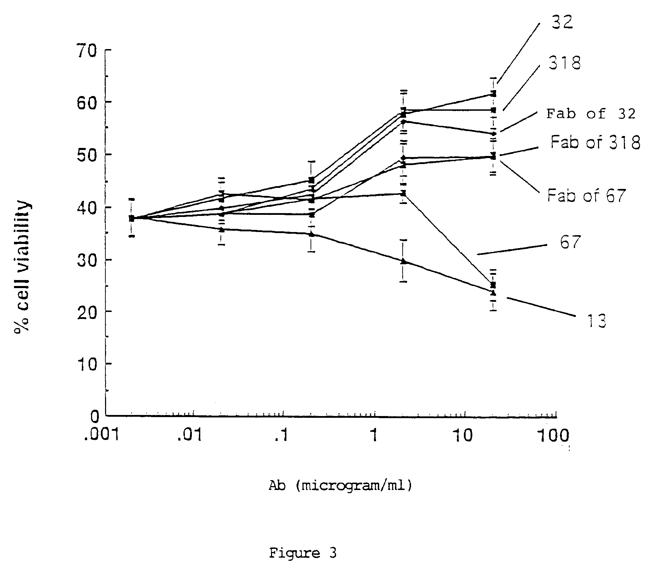

As shown in Figure 3, the protective effect of the different antibodies studied on the cytocidal effect of TNF on HeLa p75.3 cells was found to vary depending on the paricular antibody used: antibodies 32 and antiserum 318 and their Fab monovalent fragments, which protect, antibody 67, which protects as Fab monovalent fragment and enhances TNF cytotoxicity in its divalent form, and antibody 13 (which binds to the upper part of the extracellular domain of the -75-R) which enhances the cytocidal effect of TNF (p75.3 cells are HeLa cells transfected with the full length p75 TNF-R).

### EXAMPLE 3: The inhibitory effect of the group 67 and anti-stalk antibodies is independent of the expression and function of the intracellular domain of the p75 TNF-R

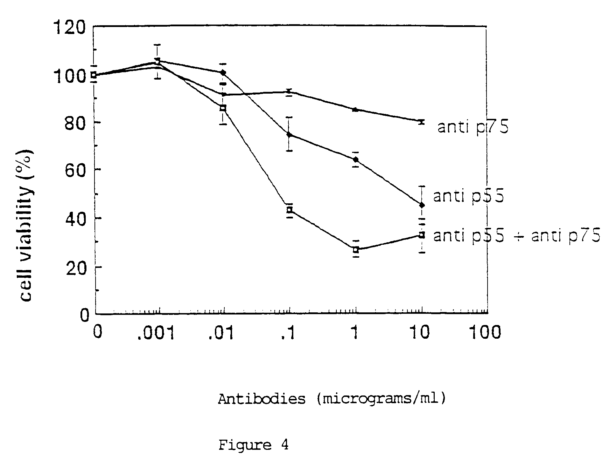

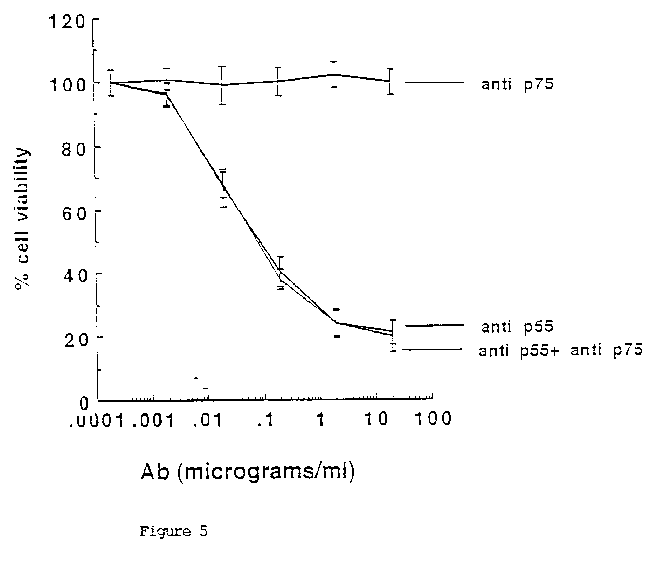

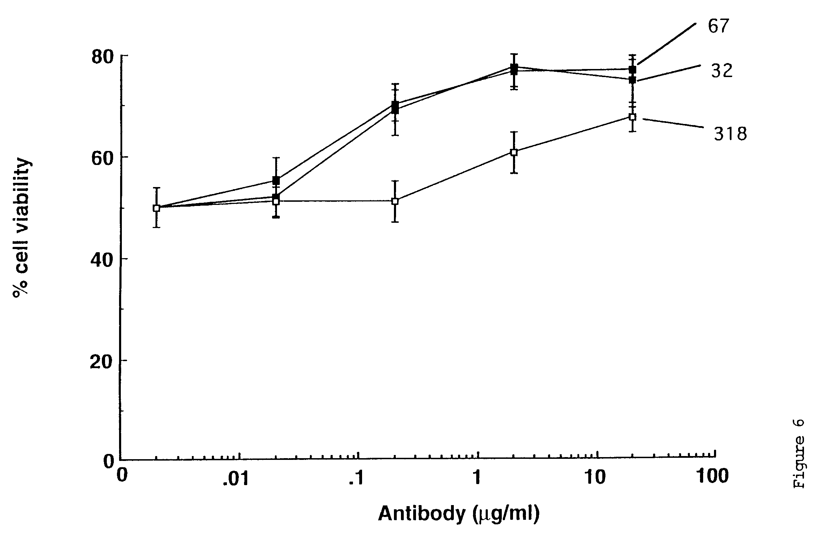

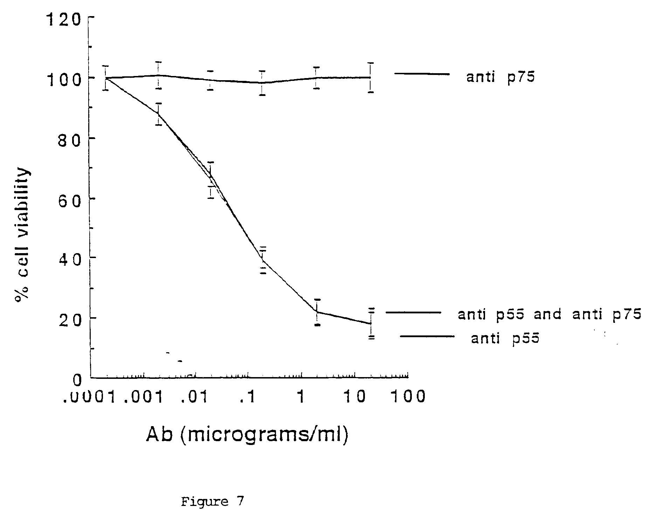

In HeLa cells which over-express the p75 TNF-R, antibodies against the upper part of the extracellular domain of the receptor have a cytocidal effect, synergistic with that of antibodies against the p55-R (Fig. 4). However, these antibodies do not have such an effect in A9 cells which express either the full-length or cytoplasmically-truncated human p75 TNF-R (Figs. 5 and 7, respectively). However, antibodies which bind to the lower part of the receptor did show inhibitory effect on TNF function even in these cells, irrespective of whether the cells expressed the full-length or the cytoplasmically truncated receptor (see Fig. 6 as well as data not shown).

### EXAMPLE 4: Effect of the various antibodies on the dissociation of TNF form p75 TNF-R

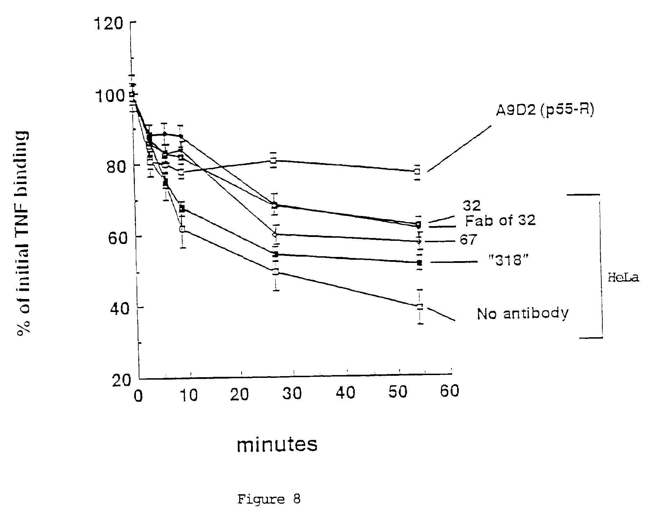

Figure 8 shows a comparison of the rate of the dissociation of TNF from the p55 TNF-R, as assessed by measuring the dissociation of radiolabeled TNF from mouse A9 cells expressing transfected human p55 TNF-R (A9D2 cells, in which over 90% of the cell-bound TNF is associated with the human p55 TNF-R) and from the HeLa p75.3 cells, in which most of the bound TNF is associated with the over-expressed p75 TNF-R. As opposed to the very slow dissociation of TNF from the p55 TNF-R, TNF dissociates rather rapidly from the p75 TNF-R.

Figure 8 also illustrates the effect of antibodies that bind to various regions at the bottom of the extracellular domain of the p75 TNF-R on the dissociation of TNF from the receptor: Antibody 32 (that binds to the "32 epitope") as well as its Fab monovalent fragments, antibody 67 - that binds to the 67 epitope, as well as antiserum 318, raised against the "stalk" region at the bottom of the extracellular domain, are all shown to impede the dissociation of TNF from the receptor.

### EXAMPLE 5

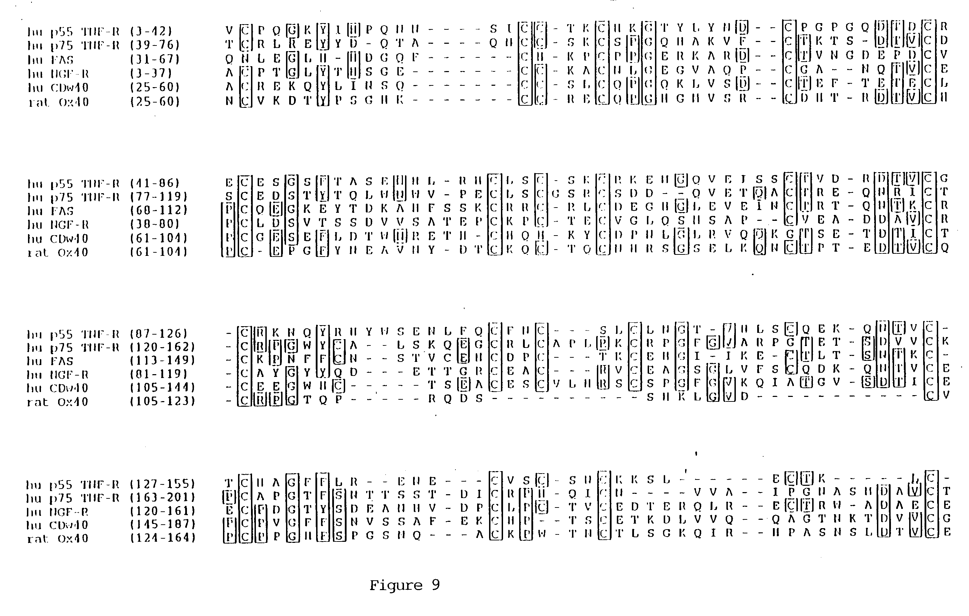

Figure 9 shows the internal cystein rich repeats in the extracellular domains of the two TNF-Rs and their alignment with the homologous repeats in the extracellular domain of the human FAS, nerve growth factor receptor (NGF) and CDw40, as well as rat Ox40. The amino acid sequences (one letter symbols) are aligned for maximal homology. The positions of the amino acids within the receptors are denoted in the left hand margin.

### Deposit Information

Hybridomas TBP-II 67 and 81 were deposited with the Collection National de Cultures de Microorganismes, Institut Pasteur (CNCM) on October 11, 1993 and assigned No.s I-1368 and I-1369, respectively.

## Claims

1. A ligand to a member of the TNF/NGF receptor family which binds either to the region or the fourth cysteine rich domain of such a receptor, or to the region between it and the cell membrane.

2. A ligand according to claim 1, wherein the fourth cysteine rich domain includes the amino acid sequence of pro-141 to thr-179 in the p75 TNF-R, or a corresponding region in another member of the TNF/NGF receptor family.

3. A ligand according to claim 2, wherein the fourth cysteine rich domain includes the amino acid sequence of Cys-163 to Pro-141 in the p75 TNF-R, or a corresponding region in another member of the TNF/NGF receptor family.

4. A ligand according to claim 1, wherein the region between the fourth cysteine rich domain and the cell membrane includes thr-179 to the end of the extracellular domain of the p75 TNF-R, or a corresponding region in another member of the TNF/NGF receptor family.

5. A ligand according to any one of the preceding claims, which comprises a ligand to a TNF-R.

6. A ligand according to claim 5, wherein the receptor is the p75 TNF-R.

7. A ligand according to any one of claims 1 to 3, including the amino acid sequence for the CDR region of the heavy chain of monoclonal antibody no. 67 (CNCM I-1368) and/or its light chain.

8. A ligand according to any one of claims 1 to 3, including the amino acid sequence for the CDR region of the heavy chain of monoclonal antibody no. 81 (CNCM I-1369) and/or its light chain.

9. A ligand according to any one of claims 1 to 3, including the amino acid sequence of an antibody raised against the fourth cysteine rich domain of a member of the TNF/NGF receptor family.

10. A ligand according to any one of claims 1 to 9, comprising a protein.

11. A ligand according to any one of claims 1 to 9, comprising a peptide.

12. A ligand according to any one of claims 1 to 9, the three-dimensional structure of which is similar as a pharmacophore to the three-dimensional structure of the protein or peptide as claimed in claims 10 and 11, and being capable of inhibiting the effect of TNF but not its binding to the TNF-R.

13. A DNA molecule encoding a ligand according to any one of claims 1 to 12, capable of expressing such a ligand.

14. A DNA molecule hybridizing to a DNA molecule according to claim 13 and capable of expressing a ligand according to any one of claims 1 to 12.

15. A replicable expression vehicle comprising a DNA molecule according to claim 13 or 14, and capable, in a transformant host cell, of expressing a ligand according to any one of claims 1 to 12.

16. A host cell transformed with the replicable expression vehicle of claim 15.

17. A prokaryotic host cell according to claim 16.

18. A eukaryotic host cell according to claim 16.

19. A process for the production of a recombinant ligand according to any one of claims 1 to 12, comprising culturing a transformed host cell according to claim 16 and recovering the recombinant ligand.

20. A pharmaceutical composition comprising a ligand according to any one of claims 1 to 12.

21. An anti-idiotypic antibody to a ligand according to any one of claims 1 to 12, capable of inhibiting the effect of TNF, but not its binding to the TNF-R.

22. Use of a ligand according to any one of claims 1 to 12 for the production of a pharmaceutical composition for increasing the inhibitory effect of a soluble receptor of the TNF/NGF receptor family.

## Patentansprüche

1. Ligand zu einem Mitglied der TNF/NGF-Rezeptorfamilie, der entweder an die Region der vierten cysteinreichen Domäne eines solchen Rezeptors oder an die Region zwischen dieser und der Zellmembran bindet.

2. Ligand nach Anspruch 1, wobei die vierte cysteinreiche Domäne die Aminosäuresequenz von Pro-141 bis Thr-179 in der p75-TNF-R oder eine entsprechende Region in einem anderen Mitglied der TNF/NGF-Rezeptorfamilie einschließt.

3. Ligand nach Anspruch 2, wobei die vierte cysteinreiche Domäne die Aminosäuresequenz von Cys-163 bis Pro-141 in der p75-TNF-R oder eine entsprechende Region in einem anderen Mitglied der TNF/NGF-Rezeptorfamilie einschließt.

4. Ligand nach Anspruch 1, wobei die Region zwischen der vierten cysteinreichen Domäne und der Zellmembran Thr-179 bis zum Ende der extrazellulären Domäne des p75-TNF-R oder eine entsprechende Region in einem anderen Mitglied der TNF/NGF-Rezeptorfamilie einschließt.

5. Ligand nach einem der vorstehenden Ansprüche, der einen Ligand für einen TNF-R umfaßt.

6. Ligand nach Anspruch 5, wobei der Rezeptor der p75-TNF-R ist.

7. Ligand nach einem der Ansprüche 1 bis 3, der die Aminosäuresequenz der CDR-Region der schweren Kette des monoclonalen Antikörpers Nr. 67 (CNCM I-1368) und/oder seiner leichten Kette einschließt.

8. Ligand nach einem der Ansprüche 1 bis 3, der die Aminosäuresequenz der CDR-Region der schweren Kette des monoclonalen Antikörpers Nr. 81 (CNCM I-1369) und/oder seiner leichten Kette einschließt.

9. Ligand nach einem der Ansprüche 1 bis 3, der die Aminosäuresequenz eines Antikörpers einschließt, der gegen die vierte cysteinreiche Domäne eines Mitglieds der TNF/NGF-Rezeptorfamilie erzeugt wurde.

10. Ligand nach einem der Ansprüche 1 bis 9, der ein Protein umfaßt.

11. Ligand nach einem der Ansprüche 1 bis 9, der ein Peptid umfaßt.

12. Ligand nach einem der Ansprüche 1 bis 9, dessen dreidimensionale Struktur als Pharmacophor ähnlich ist zur dreidimensionalen Struktur eines Proteins oder eines Peptides gemäß den Ansprüchen 10 und 11, und der zur Hemmung des Effektes von TNF, aber nicht seiner Bindung an den TNF-R fähig ist.

13. DNA-Molekül, das einen Ligand nach einem der Ansprüche 1 bis 12 codiert und zur Expression eines solchen Liganden fähig ist.

14. DNA-Molekül, das an ein DNA-Molekül nach Anspruch 13 hybridisiert und zur Expression eines Liganden nach einem der Ansprüche 1 bis 12 fähig ist.

15. Replizierbares Expressionsvehikel, das ein DNA-Molekül nach Anspruch 13 oder 14 umfaßt und fähig ist, in einer Transformanten-Wirtszelle einen Liganden nach einem der Ansprüche 1 bis 12 zu exprimieren.

16. Wirtszelle, die mit dem replizierbaren Expressionsvehikel nach Anspruch 15 transformiert ist.

17. Prokaryontische Wirtszelle nach Anspruch 16.

18. Eurkaryontische Wirtszelle nach Anspruch 16.

19. Verfahren zur Herstellung eines rekombinanten Liganden nach einem der Ansprüche 1 bis 12, das die Züchtung einer transformierten Wirtszellen nach Anspruch 16 und die Gewinnung des rekombinanten Liganden umfaßt.

20. Arzneimittel, das den Liganden nach einem der Ansprüche 1 bis 12 umfaßt.

21. Anti-idiotypischer Antikörper gegen einen Liganden nach einem der Ansprüche 1 bis 12, der zur Hemmung des Effekts von TNF, aber nicht seiner Bindung an den TNF-R fähig ist.

22. Verwendung eines Liganden nach einem der Ansprüche 1 bis 12 für die Herstellung eines Arzneimittels zur Steigerung des inhibitorischen Effekts eines löslichen Rezeptors der TNF/NGF-Rezeptorfamilie.

## Revendications

1. Ligand vis-à-vis d'un élément de la famille des récepteurs de TNF/NGF qui se lie soit à la région du quatrième domaine riche en cystéine d'un tel récepteur, soit à la région située entre celui-ci et la membrane cellulaire.

2. Ligand selon la revendication 1, le quatrième domaine riche en cystéine comprenant la séquence d'amino-acides de Pro-141 à Thr-179 dans le TNF-R p75 ou une région correspondante dans un autre élément de la famille des récepteurs de TNF/NGF.

3. Ligand selon la revendication 2, le quatrième domaine riche en cystéine comprenant la séquence d'amino-acides de Cys-163 à Pro-141 dans le TNF-R p75 ou une région correspondante dans un autre élément de la famille des récepteurs de TNF/NGF.

4. Ligand selon la revendication 1, la région située entre le quatrième domaine riche en cystéine et la membrane cellulaire comprenant Thr-179 jusqu'à la fin du domaine extracellulaire du TNF-R p75 ou une région correspondante dans un autre élément de la famille des récepteurs de TNF/NGF.

5. Ligand selon l'une quelconque des revendications précédentes, comprenant un ligand vis-à-vis d'un TNF-R.

6. Ligand selon la revendication 5, le récepteur étant le TNF-R p75.

7. Ligand selon l'une quelconque des revendications 1 à 3, comprenant la séquence d'amino-acides pour la région CDR de la chaîne lourde de l'anticorps monoclonal n° 67 (CNCM I-1368) et/ou sa chaîne légère.

8. Ligand selon l'une quelconque des revendications 1 à 3, comprenant la séquence d'amino-acides pour la région CDR de la chaîne lourde de l'anticorps monoclonal n° 81 (CNCM I-1369) et/ou sa chaîne légère.

9. Ligand selon l'une quelconque des revendications 1 à 3, comprenant la séquence d'amino-acides d'un anticorps generé contre le quatrième domaine riche en cystéine d'un élément de la famille des récepteurs de TNF/NGF.

10. Ligand selon l'une quelconque des revendications 1 à 9, comprenant une protéine.

11. Ligand selon l'une quelconque des revendications 1 à 9, comprenant un peptide.

12. Ligand selon l'une quelconque des revendications 1 à 9, dont la structure tridimensionnelle est analogue à un pharmacophore par rapport à la structure tridimensionnelle de la protéine ou du peptide selon les revendications 10 et 11, et étant capable d'inhiber l'effet du TNF mais pas sa liaison au TNF-R.

13. Molécule d'ADN codant pour un ligand selon l'une quelconque des revendications 1 à 12, capable d'exprimer un tel ligand.

14. Molécule d'ADN s'hybridant avec une molécule d'ADN selon la revendication 13 et capable d'exprimer un ligand selon l'une quelconque des revendications 1 à 12.

15. Véhicule d'expression réplicable comprenant une molécule d'ADN selon la revendication 13 ou 14 et capable, dans une cellule hôte transformée, d'exprimer un ligand selon l'une quelconque des revendications 1 à 12.

16. Cellule hôte transformée avec le véhicule d'expression réplicable de la revendication 15.

17. Cellule hôte procaryote selon la revendication 16.

18. Cellule hôte eucaryote selon la revendication 16.

19. Procédé de production d'un ligand recombiné selon l'une quelconque des revendications 1 à 12, comprenant la mise en culture d'une cellule hôte transformée selon la revendication 16 et la récupération du ligand recombiné.

20. Composition pharmaceutique comprenant un ligand selon l'une quelconque des revendications 1 à 12.

21. Anticorps anti-idiotype dirigé contre un ligand selon l'une quelconque des revendications 1 à 12, capable d'inhiber l'effet du TNF mais pas sa liaison au TNF-R.

22. Utilisation d'un ligand selon l'une quelconque des revendications 1 à 12 pour la production d'une composition pharmaceutique pour augmenter l'effet inhibiteur d'un récepteur soluble de la famille des récepteurs de TNF/NGF.
